# EUROPEAN PATENT SPECIFICATION

(11) **EP 3 873 561 B1**
(45) Date of publication and mention of the grant of the patent: **19.01.2022**
(21) Application number: 20816462.4
(22) Date of filing: 27.11.2020
(51) Int. Cl.: A61M 1/36

(54) **VENT INTERLOCK**
LÜFTUNGSVERRIEGELUNG
VERROUILLAGE D'ÉVENT

(30) Priority: 24.01.2020 GB 202001010
(43) Date of publication of application: 08.09.2021
(73) Proprietor: Spectrum Medical Ltd., Gloucestershire GL2 9QL (GB)
(72) Inventor: Turner, Stephen, East Gloucester, Gloucestershire GL2 9QL (GB)
(74) Representative: Cupitt, Philip Leslie
(86) International application number: PCT/EP2020/083736
(87) International publication number: WO 2021/148171

(56) References cited:
- WO-A1-2017/186831
- WO-A1-2019/193604
- US-A1- 2018 344 912
- US-A1- 2018 344 912

## Description

### Field of the Invention

The present invention relates to a blood flow control mechanism in a blood supply system. In particular, the present invention relates to a control system allowing the blood flow rate in the arterial blood line to be balanced with the flow rates of a venous line and a vent line.

### Background

Extracorporeal perfusion is a process in which blood from a patient is circulated outside the patient's body and re-oxygenated to be returned to the patient. More specifically, venous (oxygen-reduced) blood which has been removed from a patient via a venous line is oxygenated by exposure to an oxygenation gas in an oxygenator for supply via an arterial line back to the patient as arterial blood.

Extracorporeal perfusion is used to substitute heart and lung functionality during a medical procedure, such as open heart surgery or lung treatment. At the end of the medical procedure, extracorporeal perfusion is gradually terminated and the heart is allowed to take over blood circulation. If complications arise, extracorporeal perfusion may have to be resumed efficiently.

During open heart surgery, if any chambers of the heart have been opened and thereby exposed to air, the air can become trapped in the pulmonary veins, the left atrium and, ultimately, the left ventricle. Trapped air, or "air emboli", in these areas can be transported, via the aorta, to the patient's organs, including the brain. Air emboli entering an organ can interrupt blood flow, which can result in death of tissues dependent upon that blood flow.

To counter-act this effect, it is common prudent practice to insert a left ventricular vent into the right superior pulmonary vein, through the left atrium, across the mitral valve, and positioned to aspirate blood and any residual air from the left ventricle. The vented blood and residual air can be returned to the venous reservoir of the perfusion system where the air removed from the blood by defoaming agents, and this de-aired blood can be pumped forward to the patient.

At the end of such an operation, as the heart begins to beat and gradually fill with blood, the left ventricular vent is run slowly to evacuate any air that is in the left ventricle. This is the final opportunity to capture and remove this potentially harmful air. During this so-called "weaning" procedure, the blood level in the venous reservoir is related to the patient blood volume. In the current state of the art, the process requires the manual control of a perfusionist to ensure the safe translocation of blood from the venous reservoir into the patient's circulatory system.

GB1520364.9 by the present applicant relates to a blood flow control mechanism allowing the blood flow rate in the venous line of a blood supply system to be restricted.

WO 2019/193604 A1 relates to a medical device that is inserted into a patient's body for therapeutic and diagnostic purposes and, more particularly, relates to a ventricular decompression and assisting apparatus that includes percutaneous catheter based transvalvar ventricular venting loops for providing mechanical circulatory support for a short term.

WO 2017/186831 A1 relates to a multi or dual lumen cannulation assembly. The assembly may be configured for percutaneously placing tubes into the pulmonary artery (PA) and/or trans-septally via or in the left atrium (LA) to reduce the pressure of the right ventricle, to provide drainage of pulmonary artery blood with bypassing the lung and returning the blood to the heart, e.g. the LA, without the need for thoracotomy.

The present invention is concerned with improving the options for blood supply management in the final stages of extracorporeal perfusion.

### Summary of the Invention

In accordance with a first aspect of the present invention, there is provided a control system configured to control a plurality of blood flow rates in a perfusion system during a weaning phase as defined in claim 1.

The perfusion system comprises: a first blood line in which blood is permitted to flow at a first flow rate; a second blood line in which blood is permitted to flow at a second flow rate; an arterial blood line in which blood is permitted to flow at an arterial flow rate; and an arterial pump configured to circulate blood at the arterial flow rate in the arterial blood line. The control system may comprise a controller configured to determine the first flow rate and the second flow rate. The controller may be configured to process the first and second flow rates to determine a desired arterial flow rate. The controller may be further configured to operate in a first mode in which the controller modulates operation of the arterial pump to adjust the arterial flow rate so that the arterial flow rate matches the desired arterial flow rate.

The controller determines the first flow rate and the second flow rate. The controller may determine the first and second flow rate without receiving additional input, for example, without receiving flow values indicative of flow rate. This eliminates the need for the use of flow rate sensors. Alternatively, the controller may be configured to receive the first and second flow rates or to receive first and second flow values indicative of the first and second flow rates, respectively.

The controller processes the first and second flow rates in order to determine a desired arterial flow rate. The controller may process the first and second flow rates directly, in order to determine a desired arterial flow rate. Alternatively, the controller may process the first and second flow values, as described above, to determine a desired arterial flow value indicative of the desired arterial flow rate. Alternatively, the controller may convert the first and second flow values into the first and second flow rates of which they are respectively indicative. The controller may then process the first and second flow rates to determine a desired arterial flow rate directly.

The controller modulates the operation of the arterial pump to adjust the arterial flow rate so that the arterial flow rate matches the desired arterial flow rate. It is understood that the arterial flow rate need not precisely match the desired arterial flow rate. In other words, the arterial flow rate may only substantially match the desired arterial flow rate. For instance, there may be a permitted operating tolerance which, for the purposes of perfusion, is sufficient to consider the arterial flow rate to match the desired arterial flow rate. The arterial flow rate may be determined based on pump parameters of the arterial pump.

The arterial pump is responsive to the controller. The arterial pump may be any suitable pump, such as a peristaltic pump or roller pump, or a centrifugal pump. The arterial pump draws blood from a reservoir and brings it to a line pressure and flow rate for subsequent administration to a patient. The blood is typically pumped through an oxygenator. Other conditions, e.g. temperature, may also be adjusted prior to administration to the patient.

In embodiments, the desired arterial flow rate is determined relative to the sum of the first flow rate and the second flow rate.

For example, if the first flow rate is 3 litres per minute (Ipm) and the second flow rate is 1 Ipm, then the desired arterial flow rate may be determined relative to a flow rate of 4 Ipm. By "relative to" it is meant that the process performed by the controller uses the sum of the first flow rate and the second flow rate, e.g. 4 Ipm, in order to determine the desired arterial flow rate. For example, the controller may perform a calculation using the sum of the first flow rate and the second flow rate in order to calculate a desired arterial flow rate.

In embodiments, the desired arterial flow rate is equal to, greater than, or less than the sum of the first flow rate and the second flow rate.

If the arterial flow rate is equal to the sum of the first flow rate and the second flow rate, blood is supplied to a patient at the same rate as it is allowed to be drained. This maintains a steady amount of blood in the vascular system. Thus, extracorporeal perfusion may circulate blood at a lower rate, whereas the heart takes over the circulation of the blood in the vascular system. For instance, while the heart is stopped during a medical procedure, extracorporeal perfusion may supply and drain blood at a flow rate of 5 Ipm. The invention allows blood to be drained via the venous line, e.g., at a venous flow rate of 4.5 Ipm and blood to be aspirated via the vent line, e.g., at a vent flow rate of 0.5 Ipm, and the controller ensures that the blood is supplied at an arterial flow rate of, e.g., 5 Ipm.

The provision of a steady blood supply and drainage allows the heart performance to be monitored under better-defined conditions.

In embodiments, the first flow rate is a venous flow rate, and the first blood line is a venous line for draining blood into a reservoir.

The reservoir may be an extracorporeal venous reservoir.

In embodiments, the second flow rate is a vent flow rate, and the second blood line is a vent line for draining blood into a reservoir.

The reservoir may be the same reservoir as above. The reservoir may be the extracorporeal venous reservoir. The vent line may be a drainage line for draining blood from a patient. The vent line may be a left ventricular vent line. That is to say, the vent line may be positioned to aspirate blood from the left ventricle of the patient's heart. While the invention described herein as beneficial for use with a left ventricular vent line, it will be appreciated that the invention works equally well with vent lines located in other chambers or vessels in a patient's circulatory system. For example, the vent line may be positioned to aspirate blood from the aorta, the left atrium, the right ventricle, the pulmonary artery, or other locations in the patient's circulatory system.

In embodiments, the control system is for a perfusion system comprising a second pump configured to circulate blood at the second flow rate in the second blood line.

The second pump may be a vent pump. The second pump may be any suitable pump, such as a peristaltic pump or roller pump, or a centrifugal pump. The second pump draws blood from the vent site (e.g. the left ventricle), via the second blood line, for delivery to a reservoir. The blood drawn from the vent site by the second pump (i.e. the blood in the second blood line) may undergo a de-foaming process prior to entry to the reservoir, while in the reservoir, or after leaving the reservoir.

In embodiments, the controller is configured to determine the second flow rate based on calculations using known system parameters.

The controller may perform calculations using known system parameters in real-time to determine the second flow rate.

In embodiments, the known system parameters relate to: tube sizing parameters of the second blood line; pump sizing parameters of the second pump; and/or operational pump parameters of the second pump.

The known system parameters may be inputs provided by a user.

In embodiments, the control system comprises a first flow sensor configured to provide a first flow value indicative of the first flow rate in the first blood line.

The first flow sensor measures the first flow value. The first flow value is representative of the actual flow rate in the first blood line. The controller may be configured to receive the first flow value from the first flow sensor when determining the first flow rate. The first flow sensor may be an ultrasonic flow sensor.

In embodiments, the control system comprises a second flow sensor configured to provide a second flow value indicative of the second flow rate in the second blood line.

The second flow sensor measures the second flow value. The second flow value is representative of the actual flow rate in the second blood line. The controller may be configured to receive the second flow value from the second flow sensor when determining the second flow rate. The second flow sensor may be an ultrasonic flow sensor.

The applicant has appreciated that the use of flow rates in determining the desired arterial flow rate is beneficial over the use of reservoir level measurements. Reservoir level is also affected by other sources of fluid, for example, blood from sucker, or salvage, lines or other fluids being otherwise added to the reservoir. Such sources of fluid are not considered to be from the circulating blood volume that is to be translocated from the perfusion system to the patient (or vice versa). As such, these sources of fluid are new volumes that must be handled differently from the circulating blood volume.

For example, an additional source of fluid may introduce an additional volume into the reservoir. In such an example, it is not necessary for the perfusion process to result in this additional volume being translocated from the reservoir to the patient's vascular system. On the contrary, the presence of the additional volume may result in the overall volume present in the perfusion system being greater than the ideal, target, or desired volume of the patient's vascular system. It would therefore not be clinically desirable to translocate this additional volume into the patient's vascular system along with the patient's original blood volume. As such, the reservoir level is not an appropriate measurement for establishing the required flow rates.

This appreciation has enabled the applicant to pursue the use of flow rate measurements in order to solve this problem. By measuring flow rates (either by calculation from parameters, or using flow sensors), the arterial flow rate may be balanced by the first and second flow rates (e.g. venous and vent flow rates), as required by the perfusion requirements of the situation This would result in a steady-state being achieved with the patient's blood volume (i.e. it is unchanging) regardless of whether any fluid is added directly to the reservoir by other means.

In embodiments, the control system comprises an arterial flow sensor configured to provide an arterial flow value indicative of the arterial flow rate in the arterial line. The controller may be configured to modulate operation of the arterial pump to adjust the arterial flow rate so that the arterial flow value indicated by the arterial flow sensor matches an arterial flow value indicative of the desired arterial flow rate.

The arterial flow sensor may be a separate sensor, e.g., downstream of the pump or downstream of an oxygenator. This allows the actual flow rate to be determined, taking into account any losses that may occur between the pump and the second flow sensor.

The arterial flow sensor may be constituted by an arrangement deriving the arterial flow value from the operational parameters of the arterial pump. E.g., for a given setup, (e.g., pump speed, tube diameter, etc.), the revolutions, or strokes, per minute can be correlated with the output flow rate.

In embodiments, the control system comprises an adjustable restriction responsive to the controller. The adjustable restriction may be configured to reduce the first flow rate in the first blood line to maintain a flow rate that does not exceed a restriction threshold.

The adjustable restriction may be positioned on the first blood line.

The restriction threshold can be regarded as a maximum flow rate threshold. This may be set as a user-defined threshold. It will be understood that the restriction threshold is set at a particular flow rate level. For instance, a typical flow rate in a perfusion system - in the absence of a restriction - may be around 5 litres per minute (Ipm). In accordance with the first aspect, the restriction threshold may be set to a lower value, e.g., 2 Ipm.

The controller operates the adjustable restriction to reduce the flow rate until the first flow value, as measured by the first flow sensor, is indicative of a first flow rate at or below the restriction threshold of, e.g., 2 Ipm.

The first flow sensor may be positioned upstream or downstream of the adjustable restriction. Even if positioned upstream, the effect of a restriction on the flow rate can be accurately measured.

An adjustable restriction responsive to a flow sensor can be regarded as a closed loop control. This provides a mechanism to maintain a pre-determined flow threshold regardless of the type of tubing or the type of restriction employed. The closed loop control reduces, and practically avoids, the risk of restricting a blood line more than intended.

Hitherto, a flow rate restriction was achieved only by manually clamping a flexible tube of a blood line. Manually clamping does not allow a flow to be reduced to a chosen, pre-determined level. For illustration purposes, it is mentioned that a flexible tube has to be squeezed considerably, e.g., by more than half of its original diameter, before an effect on the flow rate becomes noticeable. The tube would have to be squeezed further to effectively reduce the flow rate. The responsive arrangement of the invention allows the level of restriction to be adjusted when the tubing is already squeezed to some extent. Due to the closed loop control, the restriction can be adjusted to a set threshold.

Furthermore, the restriction threshold may be set to a low flow level while ensuring that a minimum flow rate is maintained. This allows low restriction thresholds to be maintained in situations in which flow must not be stopped entirely.

In embodiments, the adjustable restriction comprises a gradually actuatable occlusive device.

For instance, the gradually actuatable occlusive device may be constituted by a motorised clamp acting on a flexible tube.

In embodiments where the first blood line is a venous line, the adjustable restriction is used to restrict the flow rate in the venous line. By restricting the flow rate through the venous line, the amount of blood circulating in a patient may be increased.

This functionality provides options for a better control of the extracorporeal blood supply during the end phases, or "weaning", of perfusion support. The end phases may be split into (a) initiating the end of the extracorporeal perfusion support, (b) maintaining a gradually reduced extracorporeal perfusion support to allow heart performance to be monitored, (c) if required, resuming extracorporeal perfusion, and (d) when possible, completely ceasing extracorporeal perfusion and letting the heart take over circulation.

The adjustable restriction may be positioned at an inlet of the reservoir and/or upstream of an inlet of the reservoir (in the venous line). The adjustable restriction may be configured for attachment at an inlet of the reservoir or be integral with the reservoir. The adjustable restriction and the first flow sensor may be provided as a single, integrated module. The provision of flow sensor and adjustable restriction as a single module may facilitate installation in a perfusion system.

In embodiments comprising a plurality of venous lines into the reservoir, a flow sensing arrangement capable of determining a cumulative flow value into the reservoir may be provided. The adjustable restriction may be understood as an arrangement capable of restricting the flow through each one of the one or more venous lines, to reduce the cumulative flow rate below a restriction threshold.

A single controller may be provided both to control the adjustable restriction in response to the first flow value and to modulate operation of the arterial pump. Alternatively, individual controllers may be provided, one each to control the adjustable restriction and to modulate operation of the arterial pump.

In embodiments, the control system is configured to allow the restriction threshold and the desired arterial flow rate to be set independently.

The arterial flow rate (e.g., pump performance) and the restriction threshold (e.g., the adjustable restriction) may be controlled independently.

This provides a mechanism to better control the amount of blood in the vascular system depending on the requirements of a patient. As a simplified explanation, during the end phase of extracorporeal perfusion, blood is transferred from the venous reservoir into the patient. This may be referred to as "filling" the vascular system, whereas by "filling", it is meant that the amount of blood in the vascular system is gradually increased, while correspondingly less blood is held in the extracorporeal venous reservoir.

The restriction threshold may be set via an input interface. This allows a clinician to set a restriction threshold but not alter the desired arterial flow rate, the desired arterial flow rate being determined by the controller.

In embodiments, the desired arterial flow rate may also be set via an input interface. The restriction threshold and the desired arterial flow rate may each be set via a respective input interface, thus continuing to allow a clinician to set them independently. For instance, the restriction threshold may be set to 2 Ipm, and no desired arterial flow rate may be set. Thus, the output flow rate may be governed by other clinical considerations, for example, by the controller, as described herein. Likewise, a clinician may set a desired arterial flow rate without altering the restriction threshold.

The restriction threshold and/or the desired arterial flow rate may be changed incrementally (e.g., "Increase by 0.1 Ipm" or "Reduce by 0.1 Ipm"), e.g., via a user interface with "up" and "down" buttons.

In embodiments, the controller is configured to operate in a second mode in which the controller modulates operation of the arterial pump to maintain the arterial flow rate independently of the first flow rate and/or the second flow rate.

The controller may maintain the arterial flow rate at the flow rate in the arterial blood line at the time at which the controller is switched from the first mode to the second mode. The controller mode may be switched from the first mode to the second mode and vice versa via an input interface. This allows a clinician to switch between the first mode and the second mode during a surgical procedure.

This level of control is advantageous during weaning procedures. For example, the controller can firstly be set to operate in the first mode. The controller therefore matches the arterial flow rate to the desired arterial flow rate, for example, to the sum of the venous flow rate and the vent flow rate. During the first mode, any change or fluctuation in either the vent flow rate of the venous flow rate would cause the controller to adjust the arterial flow rate to match the sum. This substantially maintains the reservoir level and a "steady-state" condition between the blood being removed from the patient (by the vent line and the venous line) and the blood being delivered to the patient (by the arterial line).

However, during weaning, it may be required to increase or decrease the volume of blood in the patient. By switching to the second mode, the arterial flow rate becomes independent of the first flow rate and/or the second flow rate. For example, the arterial flow rate may be maintained, by the controller and the arterial pump, at the flow rate at which it is currently operating (at the time of the switch to the second mode). This allows the venous flow rate and/or the vent flow rate to be adjusted.

Advantageously, in the second mode, the controller may modulate the operation of the arterial pump independently only of the first flow rate (and not the second flow rate). In such an instance, if the first flow rate in the first blood line (e.g. the vent line) experiences fluctuations then the arterial flow rate will be adjusted to maintain the steady-state (i.e. it will increase or decrease by as much as the vent flow rate). However, if the second flow rate in the second blood line (e.g. the venous line) is altered, for example by adjusting the restriction threshold, then the arterial flow rate is not adjusted. This enables, for example, the clinician to restrict the venous flow in order to increase the amount of blood circulating in the patient. Alternatively, the clinician may reduce the restriction on the venous flow in order to decrease the amount of blood circulating in the patient.

In accordance with a second aspect of the present invention, there is provided a perfusion system as defined in claim 14.

The perfusion system comprises: a first blood line in which blood is permitted to flow at a first flow rate; a second blood line in which blood is permitted to flow at a second flow rate; an arterial blood line in which blood is permitted to flow at an arterial flow rate; an arterial pump configured to circulate blood at the arterial flow rate in the arterial line; and a control system being configured to control a plurality of blood flow rates in the perfusion system during a weaning phase. The control system may comprise a controller configured to determine the first flow rate and the second flow rate. The controller may be configured to process the first and second flow rates to determine a desired arterial flow rate. The controller may be further configured to operate in a first mode in which the controller modulates operation of the arterial pump to adjust the arterial flow rate so that the arterial flow rate matches the desired arterial flow rate.

In accordance with an example not forming part of the claimed invention, there is provided a method of controlling a plurality of blood flow rates in a perfusion system.

The perfusion system comprises: a first blood line in which blood is permitted to flow at a first flow rate; a second blood line in which blood is permitted to flow at a second flow rate; an arterial blood line in which blood is permitted to flow at an arterial flow rate; and an arterial pump configured to circulate blood at the arterial flow rate in the arterial line. The method comprises the steps of: determining, by a controller, the first flow rate and the second flow rate; processing, by the controller, the first and second flow rates to determine a desired arterial flow rate; and operating the controller in a first mode, thereby modulating operation of the arterial pump to adjust the arterial flow rate so that the arterial flow rate matches the desired arterial flow rate.

Any features of the first aspect of the invention may be combined with the second aspect of the invention and/or the example method.

### Description of the Figures

Exemplary embodiments of the invention will now be described with reference to the Figures, in which:
Figure 1 shows a schematic arrangement of components of a perfusion system comprising a control system being configured to control a plurality of blood flow rates in the perfusion system during a weaning phase in accordance with embodiments of the present invention;
Figure 2 shows a schematic arrangement of components of a perfusion system comprising a control system being configured to control a plurality of blood flow rates in the perfusion system during a weaning phase in accordance with alternative embodiments of the present invention;
Figure 3 shows a schematic arrangement of components of a perfusion system comprising a control system being configured to control a plurality of blood flow rates in the perfusion system during a weaning phase in accordance with yet further alternative embodiments of the present invention;
Figure 4 shows steps of an exemplary sequence of method steps of a method for restricting the flow rate in a blood line.
Figure 5 shows steps of an exemplary sequence of method steps of a method for controlling a plurality of blood flow rates in a perfusion system in accordance with embodiments of the present invention.

### Description

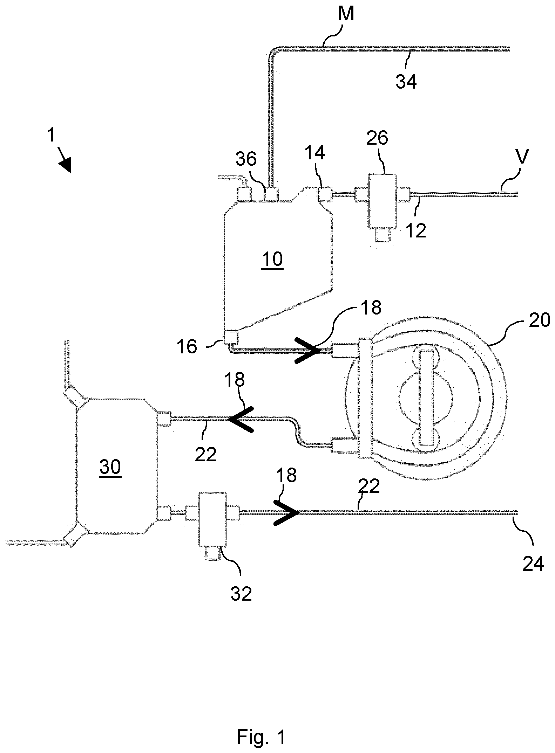

Figure 1 shows, schematically, components of a perfusion system 1. The perfusion system comprises a venous line 12 provided to receive venous blood V from a patient. Via the venous line 12, venous blood is permitted to flow into a reservoir 10 via a reservoir inlet 14. In the venous line 12, the control system comprises a venous flow sensor 26. The venous flow sensor 26 is configured to provide a flow value indicative of the flow rate in the venous line 12, and may be constituted by an ultrasonic flow meter.

The perfusion system 1 further comprises a vent line 34 provided to receive a mixture M of left ventricular blood and residual air from the left ventricle of the patient's heart. Via the vent line 34, the air-blood mixture is permitted to flow into the reservoir 10 via a reservoir inlet 36. The perfusion system 1 may further comprise a vent pump (not shown in Figure 1) for drawing blood via the vent line 34 from the patient.

De-foaming agents may be added to the blood in the reservoir to remove the residual air from the blood. Alternatively, the air-blood mixture may flow into a de-foaming system (not shown in the figure), either prior to entry to the reservoir or after exit from the reservoir. The resulting venous blood is held in the reservoir 10 at atmospheric pressure.

The venous blood may be drawn from the reservoir 10 via a reservoir outlet 16 through the main line 22 of the perfusion system. The blood is pumped by a pump 20, which may be any suitable type of pump, such as a peristaltic pump (e.g., a roller pump) or a centrifugal pump. The pump causes blood to flow through the main line 22 in a direction indicated by arrows 18, via an oxygenator 30 and towards an outlet 24 of the perfusion system 1.

At the outlet 24, the blood is in a condition for administration to a patient. For instance, the blood may have been oxygenated in the oxygenator 30, and the blood will have a flow rate and line pressure sufficient to permit safe administration to a patient. The main line 22 can therefore be considered to be an arterial line 22. The terms main line and arterial line may be used interchangeably herein. In the absence of losses, it can be assumed that the flow rate and the line pressure are determined by the pump 20. If the pump 20 generates higher throughput, the arterial flow rate is faster. Conversely, if the pump 20 generates lower throughput, the arterial flow rate is slower.

The venous line 12 and the main line 22 may be constituted by flexible tubing. The tubes may have a different length and/or diameter. The tubes may have different strength or flexibility characteristics.

In an embodiment, the venous line 12 constitutes a first blood line, the venous flow sensor 26 constitutes a first flow sensor, and the vent line 34 constitutes a second blood line.

Downstream of the reservoir 10, (in Figure 1 also downstream of the oxygenator 30), the perfusion system is provided with a main flow sensor 32. The main flow sensor 32 allows the flow rate of the blood provided towards the patient to be measured. The main flow sensor 32 may also be considered to be an arterial flow sensor. These terms may be used interchangeably herein. The flow rate towards the patient may be regarded as output, or arterial, flow rate. The flow sensor 32 is configured to provide an arterial flow value indicative of the arterial flow rate in the main blood line, i.e., of the flow rate through the outlet 24.

A controller (not shown in Figure 1) is configured to determine the venous flow rate and the vent flow rate. The controller may determine the vent flow rate based on pump parameters and known data connected with the tubing that constitutes vent line 34.

The controller is configured to receive a venous flow value indicative of the venous flow rate, as determined by the flow sensor 26. The controller may determine a venous flow rate from the venous flow value. The controller is configured to process the vent flow rate and the venous flow value to determine a desired arterial flow rate. For example, the controller may sum the venous flow rate and the vent flow rate (of which the flow values are indicative) to determine a desired arterial flow rate.

In a first mode, the controller is configured to operate the pump 20 to maintain the desired arterial flow rate. The arterial flow value may be derived from operational parameters of the pump 20. The arterial flow value may be determined by the flow sensor 32. The controller comprises decision logic to determine whether or not the arterial flow rate (as indicated by the arterial flow value) matches the desired arterial flow rate. If the arterial flow rate does not match the desired arterial flow rate, the controller will modulate the operation of pump 20 in order to match the arterial flow rate to the desired arterial flow rate. For example, if the arterial flow rate is greater than the desired arterial flow rate the controller will reduce the throughput of the pump 20 in order to reduce the arterial flow rate.

In an embodiment, the controller may be configured to receive as an input a desired offset. The desired offset indicates that the desired arterial flow rate should be greater than or less than the sum of the venous flow rate and the vent flow rate, as dictated by the value of the offset. The offset may be a specified amount (e.g. 1 Ipm) or it may be a percentage offset (e.g. 10% greater than the sum). In such an embodiment, the controller is configured to modulate the operation of the pump 20 in order to match the arterial flow rate to the desired arterial flow rate as modified by the offset.

The first mode allows, for example, the arterial flow rate to be matched to the sum of the venous flow rate with the vent flow rate. This keeps the level of blood in the reservoir 10 at a constant level, safely and automatically maintaining the balance between the venous reservoir blood volume and the patient blood volume. This relationship continues even if the venous flow rate becomes zero (e.g. if a restriction arrangement restricted venous line 12 to prevent any blood flow). At a zero venous flow rate, the pump 20 will run at precisely the same flow rate as the vent flow rate (i.e. the arterial flow rate = the vent flow rate).

In a second mode, the controller modulates operation of the arterial pump 20 to maintain the arterial flow rate independently of the first flow rate and/or the second flow rate. This allows the patient's vascular system to be filled or drained by maintaining the arterial flow rate at a set rate, while adjusting the venous flow rate and/or the vent flow rate.

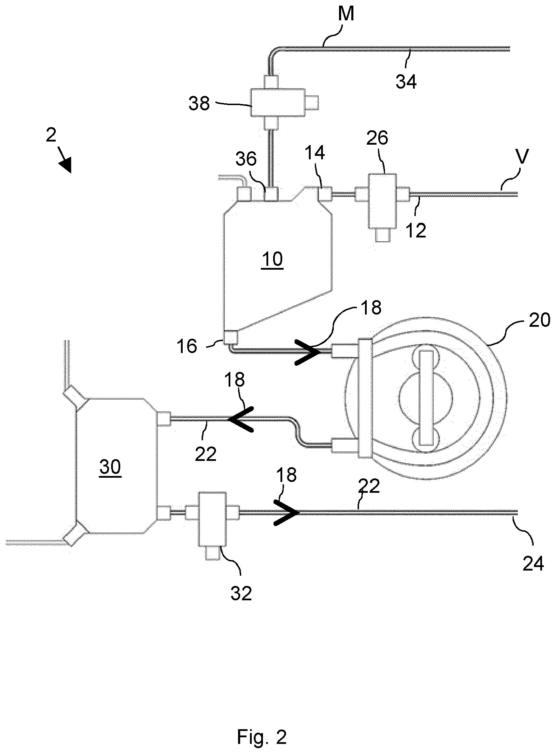

Turning to Figure 2, a schematic representation of components of a perfusion system 2 is shown. The perfusion system 2 is equivalent to perfusion system 1 except, in the vent line 34, the control system further comprises a vent flow sensor 38. Perfusion system 2 has the same operating principles as perfusion system 1, except that the controller may receive a vent flow value indicative of the vent flow rate in the vent blood line directly from the vent flow sensor 38. This means that the controller is not required to determine the vent flow rate using calculations related to known system parameters, though it may still determine in this way if so required.

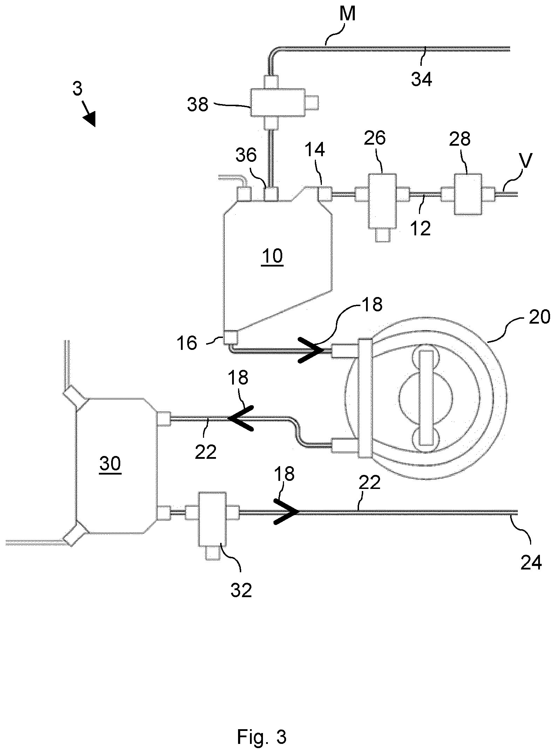

Turning to Figure 3, a schematic representation of components of a perfusion system 3 is shown. The perfusion system 3 is equivalent to perfusion system 2 except in the venous line 12, the control system further comprises a flow-restricting arrangement 28. The above description of configuration and functionality of perfusion system 2 equivalently applies to perfusion system 3. Perfusion system 3 has the additional functionality as described below. While perfusion system 3 is shown to utilise a vent flow sensor 38, it will be appreciated that perfusion system 3 need not comprise such a sensor, and could instead operate on the principles of perfusion system 1.

The flow-restricting arrangement 28 may be configured to allow the flow to be restricted gradually. For instance, the flow-restricting arrangement 28 may be constituted by a motorised clamp suitable to squeeze a flexible tube.

In an embodiment, the venous line 12 constitutes a first blood line, the venous flow sensor 26 constitutes a first flow sensor, and the flow-restricting arrangement 28 constitutes an adjustable restriction.

The motorised clamp is responsive to a controller (controller not shown in Figure 3) and allows the flow rate in the venous line to be prevented from exceeding a restriction threshold. For instance, the controller may issue a control signal to the motorised clamp to squeeze the venous line 12 until the flow rate, as determined by the flow sensor 26, no longer exceeds the restriction threshold. The controller of Figure 3 may or may not be the same as the controller of Figures 1 and/or 2.

Due to the closed loop control, it is not necessary to know by how much the tube was squeezed, or which type of equipment was used, in order to maintain the restriction threshold.

Partially clamping the flexible tube to a sufficient extent allows the flow rate in the venous line 12 to be restricted. By gradually opening the clamp, the degree of restriction of the flow rate in the venous line 12 can be reduced until there is no flow rate restriction by the flow-restricting arrangement 28.

A controller (not shown in Figure 3) is configured to receive as an input a restriction threshold to indicate the maximum flow rate through the venous line 12. For instance, the restriction threshold may be set via an interface. The restriction threshold may be set as an absolute value (e.g., 2 Ipm) or as a relative value (e.g., half of the current flow). The current flow rate may be determined by the venous flow sensor 26 or by the main flow sensor 32. For instance, the restriction threshold may be set to half the arterial flow value.

The controller is configured to receive a venous flow value indicative of the venous flow rate, as determined by the flow sensor 26. The controller comprises decision logic to determine whether or not the venous flow rate exceeds the restriction threshold. If the venous flow value does not exceed the set restriction threshold, the flow-restricting arrangement 26 is not actuated. If the venous flow value exceeds the set restriction threshold, the controller may issue a control signal to the flow-restricting arrangement 28 to increase the flow restriction until the venous flow rate no longer exceeds the restriction threshold.

After the flow-restricting arrangement has been set, the controller continues to monitor the venous flow as determined by the flow sensor 26. If, for any reason, the flow value exceeds the restriction threshold despite a previously appropriate restriction setting, the controller issues a control signal to the flow-restricting arrangement 28 to adjust the restriction threshold.

The desired arterial flow rate and the restriction threshold may each be set independently, e.g., in absolute values, via an input interface.

The controller may be configured to adjust the arterial flow rate through the outlet 24 relative to the restriction threshold in the venous line 12.

For instance, the restriction threshold and the desired arterial flow rate may be matched. The venous flow threshold may be set to 2 Ipm, and venous blood can be expected not to flow into the reservoir 10 faster than at a rate of 2 Ipm. The controller may adjust the operation of the pump 20 such that the flow rate through the outlet, as measured by the main flow sensor 32, is not more than 2 Ipm. This may be useful, for example, if vent line 34 is not in use (i.e. the vent flow rate is zero). If the vent flow rate is not zero, the desired arterial flow rate may be matched to the sum of the restriction threshold and the vent flow rate.

In the venous line 12, the actual venous flow rate is monitored by the first flow sensor 26. If, for any reason, the actual venous flow rate exceeds the threshold of 2 Ipm, the controller is configured to respond by increasing the flow restriction, until the venous flow rate is at, or below, 2 Ipm.

If the desired arterial flow rate and the restriction threshold are set independently, a change of the desired arterial flow rate will not affect the restriction threshold.

A similar process may be applied if the controller is set to match the main flow rate and the sum of the venous flow rate and the vent flow rate. For example, the venous flow restriction may be reduced by setting the venous flow threshold from 2 Ipm to 3 Ipm. If the vent line has a vent flow rate of, say, 1 Ipm, the controller may increase the pump speed until the main flow rate is 4 Ipm. Likewise, the venous flow restriction may be increased (the restriction threshold may be lowered), e.g. from 2 Ipm to 1 Ipm. Assuming the vent flow rate has remained constant, the controller may reduce the pump speed to reduce the main flow rate to 1 Ipm.

The restriction threshold may be below or above the desired arterial flow rate. This provides a control over the blood supply during the different end phases of extracorporeal perfusion.

To initiate the end of extracorporeal perfusion support, the controller can be set to operate in the second mode, and the restriction threshold may be reduced to restrict the venous flow, e.g., to 2 Ipm. At this stage, the output flow rate as determined by the pump may continue to be governed by normal perfusion requirements and the controller will no longer match the arterial flow rate to the sum of the venous flow rate and the vent flow rate. Such normal perfusion requirements may include cardiac index values and venous saturation. The arterial flow rate may be in the region of 5 Ipm. As the arterial flow rate exceeds the sum of the venous flow rate and the vent flow rate, this results in a gradual filling of the vascular system.

When the vascular system is filled to a sufficient extent (this may be determined by a physiological blood pressure), the restriction threshold may be maintained and the desired arterial flow rate may be set to match the sum of the restriction threshold and the vent flow rate, by setting the controller to operate in the first mode. The output flow rate is no longer governed exclusively by normal perfusion requirements. For instance, the desired arterial flow rate and the restriction threshold may be adjusted to such that the CVP is close to, but not exceeding 15 mmHg, and/or such that the PAD is close to, but not exceeding 20 mmHg.

The restriction threshold of the venous line and the desired arterial flow rate of the main line may be adjusted synchronously. If the heart performs satisfactorily at a perfusion flow rate of 2 Ipm, the restriction threshold and the pre-determined output flow rate may be reduced further, e.g., from 2 Ipm to 1 Ipm, to further encourage heart activity. This may affect the pressure levels, such as CVP and/or PAD. If a pressure level is too low, the desired arterial flow rate may be temporarily increased relative to the restriction threshold in order to increase the CVP or PAD value. If a pressure level is too high, the output flow rate may be temporarily decreased, and/or the restriction threshold may be partially lifted.

The restriction threshold and the desired arterial flow rate may be adjusted independently. For instance, a clinician may wish to adjust these thresholds manually according to other blood values, such as venous oxygen saturation.

If heart performance at the reduced output flow rate is insufficient, extracorporeal perfusion may be resumed by increased the output flow rate and/or by lifting the restriction threshold.

If the heart performs well with a reduced extracorporeal perfusion support, extracorporeal perfusion support may be further reduced, by setting a the desired arterial flow rate to a lower level, until a decision can be made to completely cease extracorporeal perfusion and to let the heart take over circulation.

It will be readily understood that the above-described functionality can be interchangeably applied to systems with or without an operating vent line 34. That is to say, in systems where the vent line 34 is not in use and/or the vent flow rate is zero, the arterial flow rate may be matched with or set relative to the restriction threshold or the venous flow rate. Whereas, in systems where the vent line is operational (i.e. the vent flow rate is non-zero), the arterial flow rate may be matched with or set relative to the sum of the vent flow rate and the venous flow rate, or the sum of the vent flow rate and the restriction threshold. Such logic is readily applied to the above description in relation to blood flow rate and pressure management, as well as to the filling or draining of the patient's vascular system.

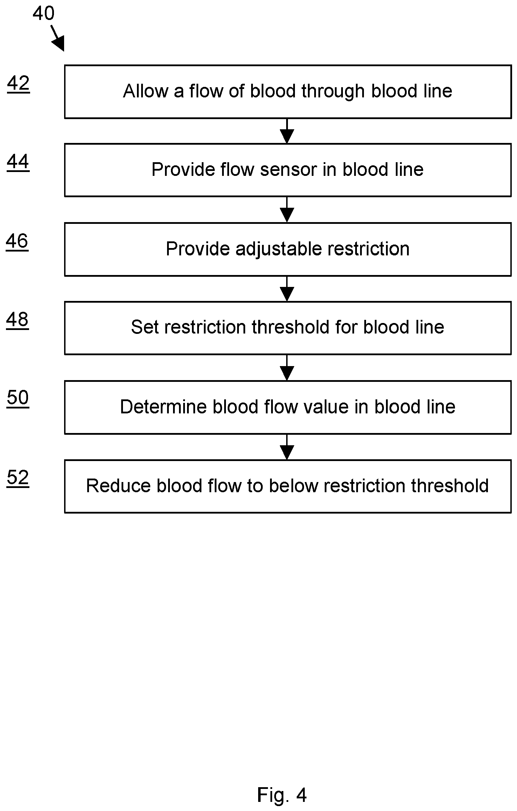

In Figure 4, steps of a control method 40 for restricting the flow rate in a blood line, such as a venous line, are shown. Such a control method may be implemented by a perfusion system such as perfusion system 3. In step 42, blood is permitted to flow through a blood line. In step 44, a blood flow sensor is provided in the blood line, to provide a blood flow value indicative of the flow rate in the blood line. In step 46, an adjustable restriction is provided to allow the flow through the blood line to be reduced. In step 48, a restriction threshold is set. The restriction threshold may be regarded as a maximum blood flow rate level. In step 50, the blood flow value (e.g., of venous blood) is determined by the blood flow sensor. In step 52, the blood flow is reduced, using the adjustable restriction, to below the restriction threshold. The restriction threshold remains responsive to the flow rate. I.e., if a blood flow value is determined to be higher than the restriction threshold, the adjustable restriction is re-adjusted to limit the flow rate to the restriction threshold.

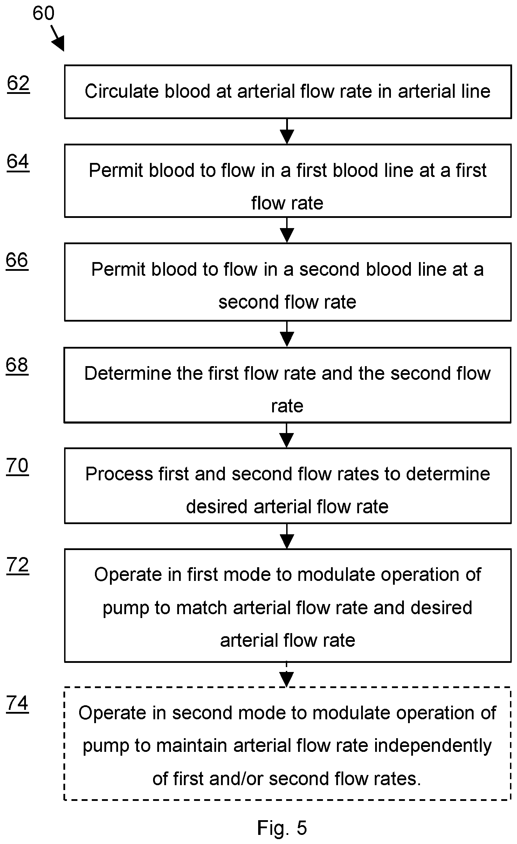

In Figure 5, steps of a control method 60 for controlling a plurality of blood flow rates in a perfusion system are shown. Such a control method may be implemented by a perfusion system such a perfusion systems 1, 2 or 3. In step 62, blood is circulated at an arterial flow rate in an arterial line. In step 64, blood flow is permitted in a first blood line (for example, a venous line) at a first flow rate. In step 66, blood flow is permitted in a second blood line (for example, a vent line) at a second flow rate. In step 68, the first and second flow rates are determined. In step 70, the first and second flow rates are processed to determine a desired arterial flow rate. Step 70 optionally comprises determining the desired arterial flow rate relative to the sum of the first and second flow values. In step 72, a controller is operated in a first mode to modulate operation of the arterial pump to adjust the arterial flow rate so that the arterial flow rate matches the desired arterial flow rate. In optional step 74, the controller is operated in a second mode to modulate operation of the arterial pump to maintain the arterial flow rate independently of the first flow rate and/or the second flow rate.

Threshold values described herein, such as the restriction threshold, the output flow rate, and pressure thresholds, may include a margin to avoid an overshooting response.

The methods disclosed herein can be performed by instructions stored on a processor-readable medium. For example, a processor-readable medium can comprise instructions that, when executed by a processor, cause the processor to perform any of the methods as previously described. Likewise, a processor-readable medium can comprise instructions that, when executed by a processor, cause the processor to implement the functionality of the control system disclosed herein. The processor-readable medium may be: a read-only memory (including a PROM, EPROM or EEPROM); random access memory; a flash memory; an electrical, electromagnetic or optical signal; a magnetic, optical or magneto-optical storage medium; one or more registers of a processor; or any other type of processor-readable medium. Alternatively, the present disclosure can be implemented as logic in hardware, firmware, software or any combination thereof. The control system may be implemented by dedicated hardware, such as one or more application-specific integrated circuits (ASICs) or appropriately connected discrete logic gates. A suitable hardware description language can be used to implement the method described herein with dedicated hardware.

It will be understood that the invention has been described above purely by way of example, and that modifications of detail can be made within the scope of the invention.

## Claims

1. A control system for a perfusion system (1), the control system being configured to control a plurality of blood flow rates in the perfusion system during a weaning phase, wherein the perfusion system comprises:
a venous blood line (12) for draining blood into a reservoir (10), wherein blood is permitted to flow in the venous blood line (12) at a venous flow rate;
a vent blood line (34) for draining a mixture comprising left ventricular blood and residual air into the reservoir (10), wherein blood is permitted to flow in the vent blood line (34) at a vent flow rate;
an arterial blood line (22) in which blood is permitted to flow at an arterial flow rate; and
an arterial pump (20) configured to circulate blood at the arterial flow rate in the arterial blood line (22), wherein the control system comprises:
a controller configured to determine the venous flow rate and the vent flow rate and to process the venous and vent flow rates to determine a desired arterial flow rate,
wherein the controller is configured to operate in a first mode in which the controller modulates operation of the arterial pump (20) to adjust the arterial flow rate so that the arterial flow rate matches the desired arterial flow rate.

2. The control system according to claim 1, wherein the desired arterial flow rate is determined relative to the sum of the venous flow rate and the vent flow rate.

3. The control system according to any of claims 1 or 2, wherein the desired arterial flow rate is equal to, greater than, or less than the sum of the venous flow rate and the vent flow rate.

4. The control system according to any of the preceding claims, wherein the perfusion system (1) comprises a vent pump configured to circulate blood at the vent flow rate in the vent blood line (34).

5. The control system according to claim 4, wherein the controller is configured to determine the vent flow rate based on calculations using known system parameters.

6. The control system according to claim 5, wherein the known system parameters relate to: tube sizing parameters of the vent blood line (34); pump sizing parameters of the vent pump; and/or operational pump parameters of the vent pump.

7. The control system according to any of claims 1 to 4, further comprising a venous flow sensor (26) configured to provide a venous flow value indicative of the venous flow rate in the venous blood line (12).

8. The control system according to any of the preceding claims, further comprising a vent flow sensor (38) configured to provide a vent flow value indicative of the vent flow rate in the vent blood line (34).

9. The control system according to any of the preceding claims, further comprising an arterial flow sensor (32) configured to provide an arterial flow value indicative of the arterial flow rate in the arterial blood line (22), wherein the controller is configured to modulate operation of the arterial pump (20) to adjust the arterial flow rate so that the arterial flow value indicated by the arterial flow sensor (22) matches an arterial flow value indicative of the desired arterial flow rate.

10. The control system according to any of the preceding claims, further comprising an adjustable restriction (28) responsive to the controller, wherein the adjustable restriction (28) is configured to reduce the venous flow rate in the venous blood line (12) to maintain a flow rate that does not exceed a restriction threshold.

11. The control system according to claim 10, wherein the adjustable restriction (28) comprises a gradually actuatable occlusive device.

12. The control system according to any of claims 10 or 11, wherein the control system is configured to allow the restriction threshold and the desired arterial flow rate to be set independently.

13. The control system according to any of the preceding claims, wherein the controller is configured to operate in a second mode in which the controller modulates operation of the arterial pump (20) to maintain the arterial flow rate independently of the venous flow rate and/or the vent flow rate.

14. A perfusion system (1) comprising:
a venous blood line (12) for draining blood into a reservoir (10), wherein blood is permitted to flow in the venous blood line (12) at a venous flow rate;
a vent blood line (34) for draining a mixture comprising left ventricular blood and residual air into the reservoir (10), wherein blood is permitted to flow in the vent blood line (34) at a vent flow rate;
an arterial blood line (22) in which blood is permitted to flow at an arterial flow rate;
an arterial pump (20) configured to circulate blood at the arterial flow rate in the arterial line (22); and
a control system according to any of claims 1-13.

15. A computer-readable medium comprising instructions that, when executed by a processor, cause a controller comprising the processor to perform a method of controlling, during a weaning phase, a plurality of blood flow rates in a perfusion system (1) comprising: a venous blood line (12) for draining blood into a reservoir (10), wherein blood is permitted to flow at a venous flow rate; a vent blood line (34) for draining a mixture comprising left ventricular blood and residual air into the reservoir (10), wherein blood is permitted to flow at a vent flow rate; an arterial blood line (22) in which blood is permitted to flow at an arterial flow rate; and an arterial pump (20) configured to circulate blood at the arterial flow rate in the arterial line (22), the method comprising the steps of:
determining, by a controller, the venous flow rate and the vent flow rate;
processing, by the controller, the venous and vent flow rates to determine a desired arterial flow rate; and
operating the controller in a first mode, thereby modulating operation of the arterial pump (20) to adjust the arterial flow rate so that the arterial flow rate matches the desired arterial flow rate.

## Patentansprüche

1. Regelungssystem für ein Perfusionssystem (1), wobei das Regelungssystem dafür konfiguriert ist, während einer Entwöhnungsphase eine Vielzahl von Blut-Fließgeschwindigkeiten in dem Perfusionssystem zu regeln, wobei das Perfusionssystem Folgendes umfasst:
eine Venenblutleitung (12) zum Ableiten von Blut in ein Reservoir (10), wobei Blut gestattet wird, in der Venenblutleitung (12) mit einer Venen-Fließgeschwindigkeit zu fließen,
eine Entlüftungsblutleitung (34) zum Ableiten eines Gemischs, das Blut aus der linken Herzkammer und Restluft umfasst, in das Reservoir (10), wobei Blut gestattet wird, in der Entlüftungsblutleitung (34) mit einer Entlüftungsfließgeschwindigkeit zu fließen,
eine Arterienblutleitung (22), in der Blut gestattet wird, mit einer Arterien-Fließgeschwindigkeit zu fließen, und
eine Arterienblutpumpe (20), die dafür konfiguriert ist, Blut mit der Arterien-Fließgeschwindigkeit in der Arterienblutleitung (22) umlaufen zu lassen, wobei das Regelungssystem Folgendes umfasst:
eine Steuerung, die dafür konfiguriert ist, die Venen-Fließgeschwindigkeit und die Entlüftungsfließgeschwindigkeit zu bestimmen und die Venen- und die Entlüftungsfließgeschwindigkeit zu verarbeiten, um eine gewünschte Arterien-Fließgeschwindigkeit zu bestimmen,
wobei die Steuerung dafür konfiguriert ist, in einem ersten Modus zu arbeiten, in dem die Steuerung den Betrieb der Arterienblutpumpe (20) moduliert, um die Arterien-Fließgeschwindigkeit so anzupassen, dass die Arterien-Fließgeschwindigkeit der gewünschten Arterien-Fließgeschwindigkeit entspricht.

2. Regelungssystem nach Anspruch 1, wobei die gewünschte Arterien-Fließgeschwindigkeit im Verhältnis zu der Summe der Venen-Fließgeschwindigkeit und der Entlüftungsfließgeschwindigkeit bestimmt wird.

3. Regelungssystem nach einem der Ansprüche 1 oder 2, wobei die gewünschte Arterien-Fließgeschwindigkeit gleich der Summe der Venen-Fließgeschwindigkeit und der Entlüftungsfließgeschwindigkeit oder größer als dieselbe oder kleiner als dieselbe ist.

4. Regelungssystem nach einem der vorhergehenden Ansprüche, wobei das Perfusionssystem (1) eine Entlüftungspumpe umfasst, die dafür konfiguriert ist, Blut mit der Entlüftungsfließgeschwindigkeit in der Entlüftungsblutleitung (34) umlaufen zu lassen.

5. Regelungssystem nach Anspruch 4, wobei die Steuerung dafür konfiguriert ist, die Entlüftungsfließgeschwindigkeit auf Grundlage von Berechnungen unter Verwendung bekannter Systemparameter zu bestimmen.

6. Regelungssystem nach Anspruch 5, wobei die bekannten Systemparameter Folgendes betreffen: Röhrenbemessungsparameter der Entlüftungsblutleitung (34), Pumpenbemessungsparameter der Entlüftungspumpe und/oder betriebliche Pumpenparameter der Entlüftungspumpe.

7. Regelungssystem nach einem der Ansprüche 1 bis 4, das ferner einen Venendurchfluss-Sensor (26) umfasst, der dafür konfiguriert ist, einen Venendurchfluss-Wert, der auf die Venen-Fließgeschwindigkeit in der Venenblutleitung (12) schließen lässt, bereitzustellen.

8. Regelungssystem nach einem der vorhergehenden Ansprüche, das ferner einen Entlüftungsdurchfluss-Sensor (38) umfasst, der dafür konfiguriert ist, einen Entlüftungsdurchfluss-Wert, der auf die Entlüftungsfließgeschwindigkeit in der Entlüftungsblutleitung (34) schließen lässt, bereitzustellen.

9. Regelungssystem nach einem der vorhergehenden Ansprüche, das ferner einen Arteriendurchfluss-Sensor (32) umfasst, der dafür konfiguriert ist, einen Arteriendurchfluss-Wert, der auf die Arterien-Fließgeschwindigkeit in der Arterienblutleitung (22) schließen lässt, bereitzustellen, wobei die Steuerung dafür konfiguriert ist, den Betrieb der Arterienblutpumpe (20) zu modulieren, um die Arterien-Fließgeschwindigkeit so anzupassen, dass der Arteriendurchfluss-Wert, der durch den Arteriendurchfluss-Sensor (22) angezeigt wird, einen Arteriendurchfluss-Wert entspricht, der auf die gewünschte Arterien-Fließgeschwindigkeit schließen lässt.

10. Regelungssystem nach einem der vorhergehenden Ansprüche, das ferner eine einstellbare Drossel (28) umfasst, die auf die Steuerung anspricht, wobei die einstellbare Drossel (28) dafür konfiguriert ist, die Venen-Fließgeschwindigkeit in der Venenblutleitung (12) zu verringern, um eine Fließgeschwindigkeit aufrechtzuerhalten, die einen Drossel-Schwellenwert nicht überschreitet.

11. Regelungssystem nach Anspruch 10, wobei die einstellbare Drossel (28) eine schrittweise zu betätigende Verschlusseinrichtung umfasst.

12. Regelungssystem nach einem der Ansprüche 10 oder 11, wobei das Regelungssystem dafür konfiguriert ist, zu ermöglichen, dass der Drossel-Schwellenwert und die gewünschte Arterien-Fließgeschwindigkeit unabhängig festgesetzt werden.

13. Regelungssystem nach einem der vorhergehenden Ansprüche, wobei die Steuerung dafür konfiguriert ist, in einem zweiten Modus zu arbeiten, in dem die Steuerung den Betrieb der Arterienblutpumpe (20) moduliert, um die Arterien-Fließgeschwindigkeit unabhängig von der Venen-Fließgeschwindigkeit und/oder der Entlüftungsfließgeschwindigkeit aufrechtzuerhalten.

14. Perfusionssystem (1), das Folgendes umfasst:
eine Venenblutleitung (12) zum Ableiten von Blut in ein Reservoir (10), wobei Blut gestattet wird, in der Venenblutleitung (12) mit einer Venen-Fließgeschwindigkeit zu fließen,
eine Entlüftungsblutleitung (34) zum Ableiten eines Gemischs, das Blut aus der linken Herzkammer und Restluft umfasst, in das Reservoir (10), wobei Blut gestattet wird, in der Entlüftungsblutleitung (34) mit einer Entlüftungsfließgeschwindigkeit zu fließen,
eine Arterienblutleitung (22), in der Blut gestattet wird, mit einer Arterien-Fließgeschwindigkeit zu fließen,
eine Arterienblutpumpe (20), die dafür konfiguriert ist, Blut mit der Arterien-Fließgeschwindigkeit in der Arterienblutleitung (22) umlaufen zu lassen, und
ein Regelungssystem nach einem der Ansprüche 1 bis 13.

15. Rechnerlesbares Medium, das Anweisungen umfasst, die, wenn sie durch einen Prozessor ausgeführt werden, bewirken, dass eine Steuerung, die den Prozessor umfasst, ein Verfahren zum Regeln, während einer Entwöhnungsphase, einer Vielzahl von Blut-Fließgeschwindigkeiten in einem Perfusionssystem (1) durchführt, wobei das Verfahren Folgendes umfasst: eine Venenblutleitung (12) zum Ableiten von Blut in ein Reservoir (10), worin Blut gestattet wird, mit einer Venen-Fließgeschwindigkeit zu fließen, eine Entlüftungsblutleitung (34) zum Ableiten eines Gemischs, das Blut aus der linken Herzkammer und Restluft umfasst, in das Reservoir (10), worin Blut gestattet wird, mit einer Entlüftungsfließgeschwindigkeit zu fließen, eine Arterienblutleitung (22), in der Blut gestattet wird, mit einer Arterien-Fließgeschwindigkeit zu fließen, und eine Arterienblutpumpe (20), die dafür konfiguriert ist, Blut mit der Arterien-Fließgeschwindigkeit in der Arterienblutleitung (22) umlaufen zu lassen, wobei das Verfahren die folgenden Schritte umfasst:
Bestimmen, durch eine Steuerung, der Venen-Fließgeschwindigkeit und der Entlüftungsfließgeschwindigkeit,
Verarbeiten, durch die Steuerung, der Venen- und der Entlüftungsfließgeschwindigkeit, um eine gewünschte Arterien-Fließgeschwindigkeit zu bestimmen, und
Betreiben der Steuerung in einem ersten Modus, dadurch Modulieren des Betriebs der Arterienblutpumpe (20), um die Arterien-Fließgeschwindigkeit so anzupassen, dass die Arterien-Fließgeschwindigkeit der gewünschten Arterien-Fließgeschwindigkeit entspricht.

## Revendications

1. Système de commande pour un système de perfusion (1), le système de commande étant configuré pour contrôler une pluralité de débits sanguins dans le système de perfusion au cours d'une phase de sevrage, dans lequel le système de perfusion comprend :
une ligne de sang veineux (12) pour drainer le sang dans un réservoir (10) dans laquelle du sang est autorisé à s'écouler dans la ligne de sang veineux (12) à un débit veineux ;
une ligne de sang d'évent (34) pour drainer un mélange comprenant du sang ventriculaire gauche et de l'air résiduel dans le réservoir (10), dans laquelle du sang est autorisé à s'écouler dans la ligne de sang d'évent (34) à un débit d'évent ;
une ligne de sang artériel (22), dans laquelle du sang est autorisé à s'écouler à un débit artériel ; et
une pompe artérielle (20) configurée pour faire circuler du sang au débit artériel dans la ligne de sang artériel (22), dans lequel le système de commande comprend :
un moyen de commande configuré pour déterminer le débit veineux et le débit d'évent et pour traiter les débits veineux et d'évent pour déterminer un débit artériel voulu ;
dans lequel le moyen de commande est configuré pour fonctionner dans un premier mode, dans lequel le moyen de commande module le fonctionnement de la pompe artérielle (20) pour ajuster le débit artériel, de sorte que le débit artériel corresponde au débit artériel voulu.

2. Système de commande selon la revendication 1, dans lequel le débit artériel voulu est déterminé par rapport à la somme du débit veineux et du débit d'évent.

3. Système de commande selon l'une quelconque des revendications 1 ou 2, dans lequel le débit artériel voulu est égal, supérieur à ou inférieur à la somme du débit veineux et du débit d'évent.

4. Système de commande selon l'une quelconque des revendications précédentes, dans lequel le système de perfusion (1) comprend une pompe de ventilation configurée pour faire circuler du sang au débit d'évent dans la ligne du sang d'évent (34).

5. Système de commande selon la revendication 4, dans lequel le moyen de commande est configuré pour déterminer le débit d'évent sur la base de calculs utilisant des paramètres de système connus

6. Système de commande selon la revendication 5, dans lequel les paramètres de système connus concernent : des paramètres de dimensionnement de tube de la ligne de sang d'évent (34) ; des paramètres de dimensionnement de pompe de la pompe de ventilation ; et/ou des paramètres opérationnels de pompe de la pompe de ventilation.

7. Système de commande selon l'une quelconque des revendications 1 à 4, comprenant en outre un capteur du flux veineux (26) configuré pour fournir une valeur de flux veineux indicative du débit veineux dans la ligne de sang veineux (12).

8. Système de commande selon l'une quelconque des revendications précédentes, comprenant en outre un capteur de flux d'évent (38) configuré pour fournir une valeur de flux d'évent indicative du débit d'évent dans la ligne de sang d'évent (34).

9. Système de commande selon l'une quelconque des revendications précédentes, comprenant en outre un capteur de flux artériel (32) configuré pour fournir une valeur de flux artériel indicative du débit artériel dans la ligne de sang artériel (22), dans lequel le moyen de commande est configuré pour moduler le fonctionnement de la pompe artérielle (20) pour ajuster le débit artériel de sorte que la valeur de flux artériel indiquée par le capteur de flux artériel (22) corresponde à une valeur de flux artériel indicative du débit artériel voulu.

10. Système de commande selon l'une quelconque des revendications précédentes, comprenant en outre une restriction ajustable (28) sensible au moyen de commande, dans lequel la restriction ajustable (28) est configurée pour réduire le débit veineux dans la ligne de sang veineux (12) pour maintenir un débit ne dépassant pas un seuil de restriction.

11. Système de commande selon la revendication 10, dans lequel la restriction ajustable (28) comprend un dispositif d'occlusion à actionnement progressif.

12. Système de commande selon l'une quelconque des revendications 10 ou 11, dans lequel le système de commande est configuré pour permettre le réglage indépendant du seuil de la restriction et du débit artériel voulu.

13. Système de commande selon l'une quelconque des revendications précédentes, dans lequel le moyen de commande est configuré pour fonctionner dans un deuxième mode, dans lequel le moyen de commande module le fonctionnement de la pompe artérielle (20) pour maintenir le débit artériel indépendamment du débit veineux et/ou du débit d'évent.

14. Système de perfusion (1), comprenant :
une ligne de sang veineux (12) pour drainer du sang dans un réservoir (10), dans lequel du sang est autorisé à s'écouler dans la ligne de sang veineux (12) à un débit veineux ;
une ligne de sang d'évent (34) pour drainer un mélange comprenant du sang ventriculaire gauche et de l'air résiduel dans le réservoir (10), dans lequel du sang est autorisé à s'écouler dans la ligne de sang d'évent (34) à un débit d'évent ;
une ligne de sang artériel (22) dans laquelle du sang est autorisé à s'écouler à un débit artériel ;
une pompe artérielle (20) configurée pour faire circuler du sang au débit artériel dans la ligne artérielle (22) ; et
un système de commande selon l'une quelconque des revendications 1 à 13.

15. Support lisible par ordinateur, comprenant des instructions qui, lors de leur exécution par un processeur, entraînent un moyen de commande comprenant le processeur, à exécuter, au cours d'une phase de sevrage, un procédé de contrôle d'une pluralité de débits sanguins dans un système de perfusion (1), comprenant : une ligne de sang veineux (12) pour drainer du sang dans un réservoir (10), dans laquelle du sang est autorisé à s'écouler à un débit veineux ; une ligne de sang d'évent (34) pour drainer un mélange contenant du sang ventriculaire gauche et de l'air résiduel dans le réservoir (10), dans laquelle du sang est autorisé à s'écouler à un débit d'évent ; une ligne de sang artériel (22) dans laquelle du sang est autorisé à s'écouler à un débit artériel ; et une pompe artérielle (20), configurée pour faire circuler du sang au débit artériel dans la ligne artérielle (22), le procédé comprenant les étapes suivantes :
détermination, par un moyen de commande, du débit veineux et du débit d'évent ;
traitement, par le processeur, des débits veineux et d'évent pour déterminer un débit artériel voulu ; et
actionnement du moyen de commande dans un premier mode, modulant ainsi le fonctionnement de la pompe artérielle (20) pour ajuster le débit artériel de sorte que le débit artériel corresponde au débit artériel voulu.
